# EUROPEAN PATENT APPLICATION

(11) **EP 2 708 606 A1**
(43) Date of publication of application: **19.03.2014**
(21) Application number: 12184548.1
(22) Date of filing: 14.09.2012
(51) Int. Cl.: C12Q 1/68

(54) **Novel biomarkers for human cervical cancer and/or HPV infection**

(71) Applicant: Rheinische Friedrich-Wilhelms-Universität Bonn, 53113 Bonn (DE)
(72) Inventor: The designation of the inventor has not yet been filed
(74) Representative: Krauss, Jan

(57) **Abstract**

The present invention relates to diagnostic biomarkers for human cervical cancer and/or HPV infection. These biomarkers help to make clinical decisions that result in improvements in the diagnosis and therapy of cervical cancer, in particular in staging thereof, and thus an early diagnosis of said cancer. Further aspects relate to diagnostic kits comprising said biomarkers, and uses thereof.

## Description

The present invention relates to diagnostic biomarkers for human cervical cancer and/or HPV infection. These biomarkers help to make clinical decisions that result in improvements in the diagnosis and therapy of cervical cancer, in particular in staging thereof, and thus an early diagnosis of said cancer. Further aspects relate to diagnostic kits comprising said biomarkers, and uses thereof.

### Background of the invention

Cervical cancer is the third most common cause of gynaecological cancer death among women in the USA (Jemal et al. 2011). Worldwide, the disease accounts for the second most gynaecological cancer death cases (Lowy and Schiller 2006). Cervical cancer is almost exclusively (99%) caused by human papilloma virus (HPV) infections. The interval between HPV infection and malignant progression takes at least 10 years (Lowy and Schiller 2006), hence cervical cancer frequency peaks at the age of 40. However, this opens a rather long period for detection and medical intervention.

At present there is no generally accepted oncogenic marker available (Bachtiary et al. 2006). Although many microarray studies have analyzed cervical cancer expression profiles, there is little overlap in the resulting gene expression signatures. This marginal concordance may result from the diverseness of the study designs or relate to the various microarray platforms and protocols used.

In addition, intratumor heterogeneity presents a different source of variation and is seldom considered. Bachtiary et al. developed a remedial measure to this situation, which is based on variance-component analysis of the genetic properties of replicate cancer biopsies (Bachtiary et al. 2006).

US 2012-129705 describes a method for predisposition and prediction of a subject to the development of cervical cancer and/or cancer precursors in an infection with the human papilloma virus (HPV) and/or for the detection of a persistent HPV infection, the method comprising the steps of obtaining a sample from the subject; and detecting at least one of the diagnostic markers or fragments thereof as listed in a Table in the sample obtained from the subject, such as TMEM45A, SERPINB5 and CDKN2A.

US 2011/301059 describes that cervical cancer cells and HPV+ head and neck cancer cells express three testis-specific genes not normally expressed in somatic cells: testicular cell adhesion molecule 1 (TCAM1), synaptonemal complex protein 2 (SYCP2) and stromal antigen 3 (STAG3). Among the three markers, TCAM1 and SYCP2 are early detection markers. Various methods for identifying a human or non-human animal as a candidate for further examination for cervical cancer, preneoplastic lesion for cervical cancer, head and neck cancer, or preneoplastic lesion for head and neck cancer are disclosed. Methods of detecting said cancers and preneoplastic lesions, methods of screening for drugs for treating said cancers and preneoplastic lesions, methods for monitoring the effectiveness of a treatment for said cancers, and methods of treating said cancers are also disclosed. Further disclosed are kits that can be used to practice the above methods.

Santin et al. (in Santin AD, Zhan F, Bignotti E, Siegel ER, Cané S, Bellone S, Palmieri M, Anfossi S, Thomas M, Burnett A, Kay HH, Roman JJ, O'Brien TJ, Tian E, Cannon MJ, Shaughnessy J Jr, Pecorelli S. Gene expression profiles of primary HPV16- and HPV18-infected early stage cervical cancers and normal cervical epithelium: identification of novel candidate molecular markers for cervical cancer diagnosis and therapy. Virology. 2005 Jan 20; 331(2):269-91) describe the use of oligonucleotide microarrays to interrogate the expression of 14,500 known genes in 11 primary HPV16 and HPV18-infected stage IB-IIA cervical cancers and four primary normal cervical keratinocyte cultures, with the goal of identifying genes with a differential pattern of expression between invasive cervical carcinomas (CVX) and normal cervical keratinocytes (NCK). Cyclin-dependent kinase inhibitor 2A (CDKN2A/p16), mesoderm-specific transcript, forkhead box M1, v-myb myeloblastosis viral oncogene homolog (avian)-like2 (v-Myb), minichromosome maintenance proteins 2, 4, and 5, cyclin B1, prostaglandin E synthase (PTGES), topoisomerase II alpha (TOP2A), ubiquitin-conjugating enzyme E2C, CD97 antigen, E2F transcription factor 1, and dUTP pyrophosphatase were among the most highly overexpressed genes in CVX when compared to NCK. Down-regulated genes in CVX included transforming growth factor beta 1, transforming growth factor alpha, CFLAR, serine proteinase inhibitors (SERPING1 and SERPINF1), cadherin 13, protease inhibitor 3, keratin 16, and tissue factor pathway inhibitor-2 (TFPI-2). The results identify several genes that are coordinately disregulated in cervical cancer, likely representing common signaling pathways triggered by HPV transformation. Moreover, the data obtained is described to provide a foundation for the development of new type-specific diagnostic and therapeutic strategies for this disease.

Wang et al. (in Wang S, Li W, Lv S, Wang Y, Liu Z, Zhang J, Liu T, Niu Y. Abnormal expression of Nek2 and β-catenin in breast carcinoma: clinicopathological correlations. Histopathology. 2011 Oct; 59(4):631-42. doi: 10.1111/j.1365-2559.2011.03941.x) describe that NIMA-related kinase 2 (Nek2) and β-catenin are important centrosome regulatory factors. They used immunohistochemistry to detect protein expression of Nek2 and β-catenin in breast cancer tissues from 60 cases of pure DCIS, 348 cases of IDC and 137 cases of concomitant DCIS with that in normal breast tissues from the same 137 concomitant DCIS patients as controls. As compared with normal tissue, expression of Nek2 and β-catenin in the cytoplasm was significantly increased in IDC and DCIS (P < 0.05), and variation in expression was also observed in different grades of IDC (P < 0.01). Nek2 cytoplasmic expression was associated with grade and tumour size (P < 0.01) in IDC, whereas β-catenin cytomembrane expression showed significant variation with grades, TNM stages, lymphoid node status, oestrogen receptor status, and molecular subtype (P < 0.05). Expression of Nek2 at the mRNA level was detected in 50 pairs of breast cancer specimens and matched normal tissues by reverse transcriptase polymerase chain reaction, and the result showed increased expression in IDC.

AURKA (Aurora Kinase A) belongs to the mitotic serine/threonine kinases family. AURKA is a cell cycle-regulated kinase which may be involved in microtubule formation and/or stabilization at the spindle pole during chromosome segregation. AURKA plays an essential role in tumourigenesis and is overexpressed in various types of cancers. AURKA is strongly expressed in the testis, colon, ovarian, prostate, neuroblastoma, breast and cervical cancer cell lines and weakly in skeletal muscle, thymus and spleen.

Inoda et al. (Inoda S, Hirohashi Y, Torigoe T, Nakatsugawa M, Kiriyama K, Nakazawa E, Harada K, Takasu H, Tamura Y, Kamiguchi K, Asanuma H, Tsuruma T, Terui T, Ishitani K, Ohmura T, Wang Q, Greene MI, Hasegawa T, Hirata K, Sato N. Cep55/c10orf3, a tumor antigen derived from a centrosome residing protein in breast carcinoma. J Immunother. 2009 Jun;32(5):474-85) describe the centrosomal protein, Cep55/c10orf3 as acting as a novel breast carcinoma-associated tumor-associated antigen. Cep55/c10orf3 mRNA was detectable in a wide variety of tumor cell lines. Expression was barely detectable in normal tissues except for testis and thymus. Monoclonal antibody # 11-55 and the Cep55/c10orf3_193(10) peptides may be useful as part of a therapeutic strategy for hormonal therapy or anti-p185 HER2/neu monoclonal antibody therapy-resistant breast carcinoma patients.

Ryu et al (Ryu B, Kim DS, Deluca AM, Alani RM. Comprehensive expression profiling of tumor cell lines identifies molecular signatures of melanoma progression. PLoS One. 2007 Jul 4;2(7):e594) then describe the unsupervised hierarchical clustering of profiling data from melanoma cell lines and melanocytes. This clustering identified two distinctive molecular subclasses of melanoma segregating aggressive metastatic tumor cell lines from less-aggressive primary tumor cell lines. Further analysis of expression signatures associated with melanoma progression using functional annotations categorized these transcripts into three classes of genes: 1) Upregulation of activators of cell cycle progression, DNA replication and repair (CDCA2, NCAPH, NCAPG, NCAPG2, PBK, NUSAP1, BIRC5, ESCO2, HELLS, MELK, GINS1, GINS4, RAD54L, TYMS, and DHFR), 2) Loss of genes associated with cellular adhesion and melanocyte differentiation (CDH3, CDH1, c-KIT, PAX3, CITED1/MSG-1, TYR, MELANA, MC1R, and OCA2), 3) Upregulation of genes associated with resistance to apoptosis (BIRC5/survivin). They conclude that tumor cell lines are a valuable resource for the early identification of gene signatures associated with malignant progression in tumors with significant heterogeneity like melanoma.

Yick-Fu Wong et al. (in Yick-Fu Wong et al. Genome-wide gene expression profiling of cervical cancer in Hong Kong women by oligonucleotide microarray International Journal of Cancer, Volume 118, Issue 10, pages 2461-2469, 15 May 2006) describe an analysis of gene expression profiles obtained from cervical cancers to find those genes most aberrantly expressed. Supervised analysis of gene expression data identified 98 and 139 genes that exhibited >2-fold upregulation and >2-fold downregulation, respectively, in cervical cancer compared to normal cervix. Several of the genes that were differentially regulated included SPP1 (Osteopontin), CDKN2A (p16), RPL39L, Clorfl, MAL, p11, ARS and NICE-1. These were validated by quantitative RT-PCR on an independent set of cancer and control specimens. Immunohistochemical staining of cancer specimens further confirmed differential expression of SPP1 in cervical cancer cells vs. nontumor cells. In addition, 2 genes, CTGF and RGS 1 were found to be upregulated in late stage cancer compared to early stage cancer, suggesting that they might be involved in cancer progression. The pathway analysis of expression data showed that the SPP1, VEGF, CDC2 and CKS2 genes were coordinately differentially regulated between cancer and normal. The study also describes several genes that may be highly attractive candidate molecular markers/targets for cervical cancer diagnosis, prognosis and therapy.

Zhang et al. (2011) describe the EMP1 tumour suppressor gene, as targeted by miR-31.

Finally, Yuasa et al. (in: Yuasa T, Yoshiki T, Isono T, Tanaka T, Okada Y Molecular cloning and expression of uroplakins in transitional cell carcinoma. Adv Exp Med Biol. 2003;539(Pt A):33-46) describe uroplakins (UPs), urothelium-specific transmembrane proteins, are as being present only in urothelia and may be good candidates as tumor markers specific for transitional cell carcinomas (TCCs). We investigated the expression of UP genes in the tissues and peripheral blood of patients with TCCs.

In their preliminary study, Bachtiary et al. modeled the genetic variability of cervical cancer tumors and estimated the reliability of potential oncogenic markers based on sample size. As the authors pointed out, their results have to be corroborated by a larger study.

However an aspect, which was not taken into account, is the variability in each separate tumor stage according to the Federation International of Gynaecology and Obstetrics (FIGO). Investigation of the different tumor stages thus allows for an even more sensitive level of prognostic capability in a potentially allocated set of oncogenic markers. Therefore the ascertainment of expression signatures of each FIGO stage increases treatment opportunities, since each stage may also require a different therapeutic approach.

Despite the above approaches, at present there are no generally accepted biomarkers for cervical cancer available. Thus, there is a continued need to provide new biomarkers for predicting the future behavior of an established cervical proliferative disorder.

Further objects and advantages will become apparent to the person of skill when reading the following more detailed description of the present invention.

In a preferred first aspect of the present invention, the invention relates to a method for diagnosing cervical cancer in a subject having an infection with the human papilloma virus (HPV) and/or for the detection of a persistent HPV infection, the method comprising detecting the expression and/or biological activity of at least one of the diagnostic markers or specifically detectable fragments thereof selected from the group consisting of PAK2, NEK2, AURKA, PRKDC, DTL, CEP55, GINS1, P11, EMP1, UPK1A, and HSPC159 in a sample obtained from said subject.

The invention is based on the new and successful attempts of the inventors to identify novel diagnostic markers for cervical cancer and/or HPV infection. Thus, in the experiments that form the basis for the present invention, a number of markers have been identified as being related to the presence and/or progression of cervical cancer in a subject having an infection with the human papilloma virus (HPV) and/or a persistent HPV infection. Many of these markers have not been known to be linked to cervical cancer.

The inventors performed biomarker discovery on a large-scale pooled microarray dataset. Eleven highly reliable markers resulting from this analysis feature cervical cancer biomarker properties. Table 1 shows the markers (biomarkers) of the present invention.

| Marker Name | Relevance* | Database Acc. number | Notes |
|---|---|---|---|
| PAK2 | 0.35 | NM_002577 | increase of the expression and/or biological activity in cancer |
| NEK2 | 0.38 | NM_002497 | increase of the expression and/or biological activity in cancer |
| AURKA | 0.37 | NM_198437 | increase of the expression and/or biological activity in cancer |
| PRKDC | 0.38 | NM_006904 | increase of the expression and/or biological activity in cancer |
| DTL | 0.35 | NM_016448 | increase of the expression and/or biological activity in cancer |
| CEP55 | 0.38 | NM_018131 | increase of the expression and/or biological activity in cancer |
| GINS1 | 0.34 | NM_021067 | increase of the expression and/or biological activity in cancer |
| P11 | 0.18 | NM_006025 | decrease of the expression and/or biological activity in cancer |
| EMP1 | 0.24 | NM_001423 | decrease of the expression and/or biological activity in cancer |
| UPK1A | 0.26 | NM_007000 | decrease of the expression and/or biological activity in cancer |
| HSPC159 | 0.26 | NM_014181 | decrease of the expression and/or |
| | | | biological activity in cancer |

| | | | |
|---|---|---|---|
| * ratio between intra stage heterogeneity (W) and total heterogeneity within stages (T) | | | |

A "diagnostic marker", "biomarker" or "marker" in the context of the present invention shall generally mean a biological molecule found in blood, other body fluids, or tissues that is a sign of a normal or abnormal process, or of a condition or disease, such as cervical cancer. Biomarkers typically differentiate an affected patient from a person without the disease. The alterations can be due to a number of factors, including germ-line or somatic mutations, transcriptional changes, and post-translational modifications. Biomarkers, which can include proteins (e.g., an enzyme or receptor), nucleic acids (e.g., DNA, mRNA, microRNA or other non-coding RNA), antibodies, and peptides, among other categories. A biomarker can also be a collection of alterations, such as gene expression, proteomic, and metabolomic signatures and/or a panel of markers. Biomarkers can be detected in the circulation (whole blood, serum, or plasma) or excretions or secretions (stool, urine, sputum, or nipple discharge), and thus easily assessed non-invasively and serially, or can be tissue-derived, and require either biopsy or special imaging for evaluation. Genetic biomarkers can be inherited, and detected as sequence variations in germ line DNA isolated from whole blood, sputum, or buccal cells, or can be somatic, and identified as mutations in DNA derived from tumor tissue (see, for example, Henry NL, Hayes DF. Cancer biomarkers. Mol Oncol. 2012 Feb 6). A "diagnostic marker", "biomarker" or "marker" in the context of the present invention can also be a gene or gene product (such as an mRNA or protein) as analyzed in the methods as described herein. Biomarkers can be indicated by any of their gene or protein name, the database accession number or DNA sequence, and thus include the complete gene, such as regulatory regions, 5'-and/or 3'-untranslated regions, introns, exons, intron/exon boundaries (including splicing relevant sequences), and cDNA sequences and/or protein (amino acid sequence) or fragments thereof that can be specifically detected, such as, for example, using specific antibody assays. In addition, mutations in the genes of these biomarkers may indicate altered functions relevant to the disease and are important for diagnostic procedures. In the context of the present invention, the expression level and/or biological activity of at least one biomarker or a panel of biomarkers according to present invention in a biological sample can be detected and/or determined (see also Hayes et al. 1996. Journal of the National Cancer Institute 88, 1456-1466).

Preferred is a method according to the present invention, wherein said detecting step is carried out by means of the determination of the gene expression of the at least one diagnostic marker in said isolated sample, such as, for example, by means of the determination of the transcripts of the diagnostic markers and/or of the proteins encoded by the diagnostic markers.

Further preferred is a method according to the present invention, wherein an increase of the expression and/or biological activity of at least one of the diagnostic markers selected from the group consisting of PAK2, NEK2, AURKA, PRKDC, DTL, CEP55, GINS1, and/or a decrease of the expression and/or biological activity of at least one of the diagnostic markers selected from the group consisting of P11, EMP1, UPK1A and HSPC159 is indicative for cervical cancer and/or for a persistent HPV infection.

In another preferred embodiment of the present invention, several of the diagnostic markers according to the present invention can be analyzed, and thus a diagnosis can be established based on (different) subsets of said biomarkers, as explained below.

Another aspect of the present invention relates to a panel (set) of biomarkers for diagnosing cervical cancer in a subject having an infection with the human papilloma virus (HPV) and/or for the detection of a persistent HPV infection, wherein said panel comprises at least two of the diagnostic markers or fragments thereof selected from the group consisting of PAK2, NEK2, AURKA, PRKDC, DTL, CEP55, GINS1, P11, EMP1, UPK1A, and HSPC159.

Particularly preferred is a set (panel) of at least one, preferably 2, 3, 4, 5, or 6 markers selected from PAK2, NEK2, AURKA, PRKDC, DTL, CEP55, and GINS1, or selected from P11, EMP1, UPK1A, and HSPC159.

Further preferred is a set (panel) of at least one, preferably 2 or 3 of the markers selected from HSPC159, DTL, and PAK2.

Further preferred is a set (panel) of at least one, preferably 2 or 3 of the markers selected from UPK1A, GINS1, CEP55, NEK2, and PRKDC.

The above sets can preferably be supplemented with the markers EMP1 and/or P11, and/or housekeeping markers as described herein.

In another aspect of the present invention said panels of markers fulfill certain statistical quality parameters for the diagnosis, such as a p-value of 0.1 and less, preferably 0.05 and less, such as 0.01 and less, and most preferred of 0.001 and less. Preferred is a panel of biomarkers for diagnosing cervical cancer in a patient, wherein said panel consists of at least two of said biomarkers or a panel according to the present invention as above, and wherein said panel has an overall p-value for diagnosing a tumor in a cervical cancer patient of 0.01, preferably of 0.001 and less.

Further preferred are panels of markers that are selected from the above markers and, for example, can be used to identify risk groups, for example, groups of low or high risk. The low risk group could be spared of therapy, and the high risk groups would be chosen to receive certain therapies e.g. chemotherapy or others. The classification of tumors may aid to design more aggressive or specific therapies which are able to overcome, e.g., a resistant state of such tumors, or can be used to design strategies that prevent the development of chemotherapy resistance (as well).

Based on the analysis of the biomarkers as identified following a patient-unspecific clinical treatment scheme, another aspect of the method according to the present invention then relates to a step of selecting patient- and/or stage of cancer-specific biomarkers, and thus generating of a profile (or panel) of markers according to the present invention, that are of particular relevance for the individual patient and/or stage of cancer, or for a (sub)group of patients (cohort) of certain patients. This selection of markers also has the advantage that not all markers have to be analyzed all the time, without that the (statistical and/or clinical) relevance of the analysis regarding the stage of cancer and/or patient is insufficient.

Further preferred is a method according to the present invention, wherein said diagnosing furthermore comprises determining the expression and/or biological activity of a reference marker, such as, for example a housekeeping gene selected from at least one of N-acylsphingosine amide hydrolase 1, HIG1 domain family member 1A (hypoxia-inducible gene 1), and stress-associated endoplasmic reticulum protein 1. The reference marker can serve as an internal control in order to determine an increase or decrease of the expression and/or biological activity of the other biomarkers as analyzed.

Yet another aspect of the present invention then relates to a method according to the present invention, wherein said diagnosing furthermore comprises determining the stage of the cervical cancer and/or persistent HPV infection, based on said at least one diagnostic marker. It was found that some of the markers are up-regulated in stages of cervical cancer, preferably the very early stages of the cervical cancer, such as IB, IB1 and/or IBII, and some are down-regulated upon a transition from non-cancer ("NC") to cervical cancer stages, preferably the very early stages of the cervical cancer, such as IB, IB1 and/or IBII. Stages are determined based on the FIGO score.

In the methods of the present invention, in general the biomarkers can be detected and/or determined using any suitable assay. Detection is usually directed at the qualitative information ("marker yes-no"), whereas determining involves analysis of the quantity of a marker (e.g. expression level and/or activity). Detection is also directed at identifying mutations that cause altered functions of individual markers. The choice of the assay(s) depends on the parameter of the marker to be determined and/or the detection process. Thus, the determining can preferably comprise a method selected from subtractive hybridization, microarray analysis, DNA sequencing, qPCR, ELISA, enzymatic activity tests, cell viability assays, for example an MTT assay, phosphoreceptor tyrosine kinase assays, phospho-MAPK arrays and proliferation assays, for example the BrdU assay, proteomics, and mass spectrometry.

Preferably, the methods according to the present invention are also amenable to automatization, and, for example, the activity and/or expression is assessed in an automated and/or high-throughput format. Usually, this involves the use of chips and respective machinery, such as robots.

In the context of the present invention, any biological sample obtained from said cancer patient or subject to be diagnosed can be used, as long as it contains (or is presumed to contain) at least one of the biomarker(s) to be used in the analysis or screen. Preferably, said sample is selected from tumor tissue (preferably cervical tumor or metastases), biopsies, whole blood, peripheral blood, or fractions thereof, serum, buffy coat, lymphatic fluid, urine, bone marrow, EDTA plasma, heparinized plasma, citrate plasma, heparinized whole blood, and frozen samples thereof, such as frozen heparinized whole blood. More preferably, said sample is a tissue or body fluid sample of a human, such as, for example, a biopsy, a cervical cell sample and/or a Papanicolaou smear.

Yet another aspect of the present invention then relates to a method for identifying an anti-cervical cancer and/or anti-HPV infective agent, comprising the steps of a) contacting a sample obtained from a subject having cervical cancer and/or a persistent HPV infection with at least one compound which potentially modifies the expression and/or biological activity of at least one diagnostic marker selected from the group consisting of PAK2, NEK2, AURKA, PRKDC, DTL, CEP55, GINS1, P11, EMP1, UPK1A, and HSPC159, and b) identifying a compound that modifies the expression and/or biological activity of said at least one diagnostic marker.

Preferably, this method identifies an anti-cervical cancer agent or an agent against HPV infection, based on a sample derived from a patient that suffers from a cervical cancer and/or an HPV infection, or a progressing or relapsing cancer. The biomarker or the panel of biomarkers according to the present invention can be patient- and/or cancer-specific, and has optionally been generated as above. Thus, the anti-cervical cancer and/or HPV-infection agent will be of particular relevance for the individual patient and/or cancer, or for a (sub)group of patients (cohort) of certain patients.

Preferred is a method according to the present invention, wherein said compound as identified (screened) is selected from small chemical molecules, peptides, antibodies, and short interfering RNAs. Identification and screening can be done using respective methods known in the art, preferably using recombinantly produced proteins of the biomarkers, and/or recombinant cell models. For this, the biomarkers can be labeled, e.g. using chemical dyes or fluorescent markers. Also enzymes can be used, optionally in the form of fusions with the biomarker to be screened. Preferably, said method is also amenable to automatization, and said screening is preferably assessed in an automated and/or high-throughput format.

Preferred is a method according to the present invention, wherein said compound is selected from a phospholipase inhibitor such as, for example, darapladib, and/or an angiogenic inhibitor, such as, for example, angiocidin, or chemical derivatives thereof.

Another aspect of the present invention then relates to a method for producing an anti-cancer pharmaceutical preparation, wherein said compound as identified (screened) is further formulated into a pharmaceutical preparation by admixing said (at least one) compound as identified (screened) with a pharmaceutically acceptable carrier. Pharmaceutical preparations can be preferably present in the form of injectibles or tablets or capsules. Another aspect of the present invention then relates to an anti-cancer pharmaceutical preparation as prepared according to the invention.

A "pharmaceutically acceptable carrier" as used herein refers to a pharmaceutically acceptable material, composition, or vehicle that is involved in carrying or transporting a compound of interest from one tissue organ, or portion of the body to another tissue, organ, or portion of the body. Such a carrier may comprise, for example, a liquid, solid, or semi-solid filler, solvent, surfactant, diluent, excipient, adjuvant, binder, buffer, dissolution aid solvent, encapsulating material, sequestering agent, dispersing agent, preservative, lubricant disintegrant, thickener, emulsifier, antimicrobial agent antioxidant, stabilizing agent coloring agent, or some combination thereof. Each component of the carrier must be "pharmaceutically acceptable" in that it must be compatible with the other ingredients of the composition and must be suitable for contact with any tissue, organ, or portion of the body that it may encounter, meaning that it must not carry a risk of toxicity, irritation allergic response, immunogenicity or any other complication that excessively outweighs its therapeutic benefits. Examples of pharmaceutically acceptable carriers for use in the presently disclosed pharmaceutical compositions include, but are not limited to, diluents such as microcrystalline cellulose or lactose (e.g., anhydrous lactose, lactose fast flo), binders such as gelatin, polyethylene glycol, wax, microcrystalline cellulose, synthetic gums such as polyvinylpyrrolidone, or cellulosic polymers such as hydroxypropyl cellulose (e.g., hydroxypropyl methylcellulose (HPMC)), lubricants such as magnesium stearate, calcium stearate, stearic acid, or microcrystalline cellulose, disintegrants such as starches, cross-linked polymers, or celluloses (e.g., croscarmellose sodium (CCNa), fillers such as silicon dioxide, titanium dioxide, microcrystaliine cellulose, or powdered cellulose, surfactants or emulsifiers such as polysorbates (e.g., Polysorbate 20, 40 60, or 80, Span 20, 40, 60, 65, or 80) antioxidant agents such as butylated hydroxyanisole (BHA), butylated hydroxytoluene (BHT), propyl gallate, or ascorbic acid (either free acid or salt forms thereof), buffers such as phosphate or citrate buffers, sequestering agents such as ethylenediaminetetraacetic acid (EDTA), ethylene glycol tetraacetic acid (EGTA), or edetate disodium, dispersing agents such as sodium carboxymethylcelluose, hydroxypropyl methylcellulose, povidone, or polyvinylpyrrolidone, dissolution aids such as calcium carbonate, and excipients such as water, saline, dextrose, glycerol, or ethanol, citric acid, calcium metabisulfite, lactic acid, malic acid, succinic acid or tartaric acid.

A preferred aspect of the present invention then relates to darapladib and/or angiocidin for the treatment of cervical cancer and/or HPV infection.

The inventors' findings furthermore establish darapladib (N-(2-diethylaminoethyl)-2-[2-[(4-fluorophenyl)methylsulfanyl]-4-oxo-6,7-dihydro-5H-cyclopenta[d]pyrimidin-1-yl]-N-[[4-[4-(trifluoromethyl)phenyl]phenyl]methyl]acetamide) as a novel (adjuvant) treatment option for cervical cancer therapy, since the inventors repeatedly found gene expression signatures which point to an efficiency of the clinical treatment using darapladib (Wilensky et al. 2008). These results present significant finding for cervical cancer patients. The lipoprotein-associated phospholipase A2 (Lp-PLA2) inhibitor darapladib represses 24 genes of which 20 are induced in the data of the invention. Wilensky et al. showed that darapladib reduces lesions in atherosclerosis and exerts also anti-inflammatory action (Wilensky et al. 2008). The use of darapladib for the treatment of cervical cancer and/or HPV infections has not been described or suggested. Without wanting to be bound by theory, incident HPV infections either manifest as cervical intraepithelial neoplasia (CIN) lesions or become undetectable within 36 months (Insinga et al. 2005). Therefore the formation of lesions in cervical cancer can be reduced by darapladib and this should lead to clearing the HPV infection, and the treatment of cancer. Furthermore, due to the reduction of the lesions, the risk for dangerous multi-infections can be reduced or avoided.

The inventors' findings also establish the therapy of cervical cancer using the angiogenic inhibitor angiocidin. Gaurnier et al. found that angiocidin, the protein product of PSMD4, induces in total 59 genes in THP-1 cells, which subsequently transform the cells into anti-tumoral, MMP9 secreting monocytes (Gaurnier-Hausser et al. 2008). The inventors found 23 of the genes significantly induced in stage IB1, IIA and IIB. A repeated activation of these genes seems in response to virus life cycle signals activating the immunological defense mechanism for viral clearance. The use of angiocidin for the treatment of cervical cancer and/or HPV infections has not been described or suggested. Without wanting to be bound by theory, one might hypothesize that this is due to the influence of virus proteins, as seen in literature the virus protein E6 is able to alter host physiology to great extent, i.e. by degrading p53 (Rosty et al. 2005).

Another aspect of the present invention then relates to an anti-cancer pharmaceutical preparation, comprising darapladib and/or angiocidin together with a pharmaceutically acceptable carrier. Pharmaceutical preparations can be preferably present in the form of injectibles or tablets or capsules, and preferably contain a therapeutically effective amount of darapladib and/or angiocidin.

Another aspect of the present invention then relates to a method for treating and/or preventing cervical cancer in a patient having an infection with the human papilloma virus (HPV) and/or a persistent HPV infection, comprising the steps of a) performing a method according to the present invention as above, and b) providing a suitable anti-cancer and/or anti-HPV infection treatment to said patient, wherein said treatment is based, at least in part, on the results of the method according to the present invention. Optionally, steps a) and b) of the method can be repeated. The treatment thus is based, at least in part, on the diagnostic markers or specifically detectable fragments thereof according to the present invention selected from the group consisting of PAK2, NEK2, AURKA, PRKDC, DTL, CEP55, GINS1, P11, EMP1, UPK1A, and HSPC159 in a sample obtained from said patient.

Usually, the treatment begins/continues as long as an increase of the expression and/or biological activity of at least one of the diagnostic markers selected from the group consisting of PAK2, NEK2, AURKA, PRKDC, DTL, CEP55, GINS1, and/or a decrease of the expression and/or biological activity of at least one of the diagnostic markers selected from the group consisting of P11, EMP1, UPK1A and HSPC159 can be detected, which is/are indicative for cervical cancer and/or for a persistent HPV infection.

"Treatment" shall mean a reduction and/or amelioration of the symptoms of the disease, here cervical cancer. An effective treatment achieves, for example, a shrinking of the mass of a tumor and the number of cancer cells. A treatment can also avoid (prevent) and reduce the spread of the cancer, such as, for example, affect metastases and/or the formation thereof. A treatment may be a naïve treatment (before any other treatment of the cancer had started), or a treatment after the first round of treatment (e.g. after surgery or after a relapse or following the development of resistance, such as, for example, multidrug-resistant (MDR) cancer), such as, for example, an adjuvant treatment. The treatment can also be a combined treatment, involving, for example, chemotherapy, surgery, and/or radiation treatment. In the context of the present invention, a "therapy" shall mean any therapeutic intervention that reduces, eradicates, and/or slows down the growth of cancer cells in a cervical cancer patient. The cancer cells to be treated can further include metastasized cells, i.e. tumor cells outside of the cervical tissues based on the initial cervical cancer.

Preferred is a method according to the present invention, further comprising selecting a cancer-specific and/or patient-specific therapy for the treatment of cervical cancer based on the results of the method according to the invention, preferably by improving the clinical endpoints. In practice, this is done by providing a treatment and/or treatment scheme for the patient that has a positive effect on the analysis as performed based on the biomarker(s) of the invention, in particular on the patient-specific panel of markers as identified according to the above. A positive effect can be assumed once the expression and/or activity of the markers as identified as being relevant is modified, for example in that the over-expression of one or more of the markers PAK2, NEK2, AURKA, PRKDC, DTL, CEP55, GINS1 is reduced, and/or of at least one of the diagnostic markers selected from the group consisting of P11, EMP1, UPK1A and HSPC159 is increased.

Preferred is a method according to the present invention, wherein said method also comprises administering to said patient a therapeutically effective amount of the anti-cancer and/or anti-HPV agent as identified with a method according to the invention as above, preferably darapladib and/or angiocidin.

A "therapeutically effective amount" of a composition as used herein is an amount of a composition that produces a desired therapeutic effect in a subject, such as treating a target condition, such as cervical cancer and/or HPV infection. The precise therapeutically effective amount is an amount of the composition that will yield the most effective results in terms of therapeutic efficacy in a given subject. This amount will vary depending upon a variety of factors, including but not limited to the characteristics of the therapeutic composition (including, e.g. activity, pharmacokinetics, pharmacodynamics, and bioavailability) the physiological condition of the subject (including, e g age, body weight, sex, disease type and stage, medical history, general physical condition, responsiveness to a given dosage, and other present medications), the nature of the pharmaceutically acceptable carrier or carriers in the composition, and the route of administration. One skilled in the clinical and pharmacological arts will be able to determine a therapeutically effective amount through routine experimentation, namely by monitoring a subject's response to administration of a composition and adjusting the dosage accordingly. For additional guidance, see, e.g., Remington The Science and Practice of Pharmacy, 21st Edition, Univ of Sciences in Philadelphia (USIP), Lippincott Williams & Wilkins, Philadelphia, PA, 2005 and Goodman & Gilman's The Pharmacological Basis of Therapeutics, 11th Edition, McGraw-Hill, New York, NY, 2006, the entire disclosures of which are incorporated by reference herein.

Another aspect of the present invention then relates to a method for monitoring the treatment of a subj ect having cervical cancer and/or a persistent HPV infection, comprising the steps of a) providing an anti-cervical cancer and/or anti-HPV infective treatment to said subject as described herein, and b) performing a diagnosis according to a method according to the present invention on a sample obtained from said subject, and c) optionally, repeating steps a) and b), if required. Preferred is the method according to the present invention, wherein said treatment comprises the administration of a phospholipase inhibitor, such as, for example, darapladib, and/or an angiogenic inhibitor, such as, for example, angiocidin.

Further preferred is a method according to the present invention, wherein said method also comprises monitoring said treatment of said patient as above, comprising repeating said method according to the invention as above during the treatment. A positive effect can be assumed once the expression and/or activity of the markers as identified as being relevant for the patients cancer and/or HPV infection is modified, for example in that the over-expression of one or more of the markers is reduced.

Based on the analysis of the biomarkers as identified following a patient-unspecific clinical treatment scheme, another aspect of the method according to the present invention then relates to a step of selecting patient- and/or cancer-specific biomarkers, and thus generating of a profile (or panel) of markers according to the present invention, that are of particular relevance for the individual patient and/or cancer, or for a (sub)group of patients (cohort) of certain patients.

Thus, preferred is a method according to the present invention, further comprising the step of generating an expression profile and/or activity profile based on said diagnostic markers, wherein said profile consists of diagnostic markers that are indicative for the overall survival and/or the disease-free survival of said cancer patient, and/or are cancer and/or patient specific, and/or consists of markers that predict response to specific treatments, in particular patient-specific treatments. This selection of markers also has the advantage that not all markers have to be analyzed all the time, without that the (statistical and/or clinical) relevance of the analysis regarding the cancer and/or patient is insufficient.

Another aspect of the present invention then relates to a diagnostic kit comprising materials for performing a method according to the present invention in one or separate containers, preferably comprising at least one biomarker-specific antibody, optionally together with auxiliary agents and/or instructions for performing said method.

Further preferred are diagnostic kits ("panel kits") relating to specific marker combinations that are selected from the above markers. These panels can be, for example, biomarkers that can be used to identify risk groups, for example, groups of low or high risk. The kit would be used to identify a low risk group that would be spared of therapy, and a high risk group would be chosen to receive certain therapies e.g. chemotherapy or others. Furthermore, the classification of tumors with highly significant biomarkers may aid to design more aggressive or specific therapies which are able to, for example, overcome the resistant state of such tumors, or can be used to design strategies that prevent the development of chemotherapy resistance (as well).

Preferably, said kit for carrying out the methods according to the present invention can contain at least one isolated or purified oligonucleotide, the oligonucleotide being selected from at least one of the following: (a) oligonucleotides that hybridize under stringent conditions to the sequences defined in table 1 as above, or specific fragments thereof; and (b) oligonucleotides that have a sequence homology of at least 80%, preferably of at least 85%, 90%, 95% or 98% to the oligonucleotides defined in (a) and with which the diagnostic markers can likewise be detected.

Another aspect of the present invention then relates to a diagnostic kit as above, further comprising materials for determining the expression and/or biological activity of a reference marker, such as, for example a housekeeping gene selected from at least one of N-acylsphingosine amide hydrolase 1, HIG1 domain family member 1A (hypoxia-inducible gene 1), and stress-associated endoplasmic reticulum protein 1. The reference marker can serve as an internal control in order to determine an increase or decrease of the expression and/or biological activity of the other biomarkers as analyzed.

The present inventors preformed a functional investigation of gene expression profiles of cervical cancer stages and identified potential clinically relevant diagnostic biomarkers. For this purpose, gene expression profiles of FIGO staged cohorts were recruited from available microarray studies. After quality checks and normalization gene expression profiles of 126 patients were obtained for further processing by variance-component analysis and GSEA. This approach avoids the analysis of highly variable genes, which tend to result in speculative expression signatures. Instead, a conservative approach explores reliable biomarkers genes, which are constitutively expressed.

Using variance-component analysis, the inventors identified eleven genes of diagnostic biomarkers for use in cervical cancer. Seven markers are constitutively up-regulated in all cervical cancer stages. Among these genes are four kinases, namely PAK2, NEK2, AURKA and PRKDC. Furthermore the inventors found the induction of the ligase DTL, and a gene which encodes the centrosomal protein 55 (CEP55). Additionally, up-regulated gene expression of GINS1 was identified, which is essential for DNA replication in yeast and xenopus eggs (Ueno et al. 2005).

On the other hand four genes were found which are frequently down-regulated in cervical cancer patients. Another down-regulated gene is UPK1A, which encodes a cell surface protein and HSPC159 which encodes a galectin related protein.

In conclusion, since these biomarker genes are derived from actual patient data and are of low variability they present excellent diagnostic candidates.

GSEA of the variance-component processed patient matrix comprised 9.873 markers, which had a W/T ratio of less than 0.75. It is striking that the results generated by the analysis of the inventors did overlap with three cervical cancer studies from the recent literature (Rosty et al. 2005; Pyeon et al. 2007; Slebos et al. 2006).

Additionally, there is also overlap in the resulting gene expression signatures with two other virus-related gene signatures from HBV and HCMV studies (Browne et al. 2001; Wieland et al. 2004). These results acknowledge the present conservative approach, since as seen in literature other approaches tend to produce only marginal overlap in the resulting gene expression signatures (Wolfer and Ramaswamy 2011).

The inventors' findings furthermore support darapladib as a novel adjuvant treatment option for consideration in cervical cancer therapy, since the inventors repeatedly found gene expression signatures which point to darapladib (Wilensky et al. 2008). These results present significant finding for cervical cancer patients. The lipoprotein-associated phospholipase A2 (Lp-PLA2) inhibitor darapladib represses 24 genes of which 20 are induced in the data of the invention. Wilensky et al. showed that darapladib reduces lesions in atherosclerosis and exerts also anti-inflammatory action (Wilensky et al. 2008). The use of darapladib for the treatment of cervical cancer and/or HPV infections has not been described or suggested. Without wanting to be bound by theory, incident HPV infections either manifest as cervical intraepithelial neoplasia (CIN) lesions or become undetectable within 36 months (Insinga et al. 2005). Therefore the formation of lesions in cervical cancer can be reduced by darapladib and this should lead to clearing the HPV infection. Furthermore, due to the reduction of the lesions, the risk for dangerous multi-infections can be reduced or avoided.

The inventors' findings also support the therapy of cervical cancer using the angiogenic inhibitor angiocidin. Gaurnier et al. found that angiocidin, the protein product of PSMD4, induces in total 59 genes in THP-1 cells, which subsequently transform the cells into anti-tumoral, MMP9 secreting monocytes (Gaurnier-Hausser et al. 2008). The inventors found 23 of the genes significantly induced in stage IB1, IIA and IIB. A repeated activation of these genes might be in response to virus life cycle signals activating the immunological defense mechanism for viral clearance. The use of angiocidin for the treatment of cervical cancer and/or HPV infections has not been described or suggested. Without wanting to be bound by theory, one might hypothesize that this is due to the influence of virus proteins, as seen in literature the virus protein E6 is able to alter host physiology to great extent, i.e. by degrading p53 (Rosty et al. 2005).

The analysis of cervical cancer patient gene expression data presents a novel perspective on HPV mediated transcription processes. There is a growing magnitude of published gene expression signatures and biological pathways, which in future could provide a better basis for understanding of how HPV evades the immune response permanently. This knowledge may have implications for other tumorigenic viruses also. Thus established gene expression signatures may harbor the next major treatment opportunity in cancer therapy.

The invention shall now be further described in the following examples with reference to the accompanying Figures, nevertheless, without being limited thereto. For the purposes of the present invention, all references as cited herein are incorporated by reference in their entireties. In the Figures,
Figure 1 shows the heatmap of the biomarker genes according to the invention that are constitutively expressed in all FIGO stages.
Figure 2 shows the gene set enrichment analysis-derived "Wilensky" response to darapladib signature. The relevant markers are PTAFB, GM2A, NPL, DENND2D, CCR", CD4, BIN2, ARRB2, CYBB, CCR1, APOE, UCP2, EYL2A, PLA2G7, CCL5, CD68, CHI3L1, CD48, CTSS, and EYL2B.
Figure 3 shows the gene set enrichment analysis-derived "Gaurnier" PSMD4 targets signature. The relevant markers are CXCL11, CCL19, CXCL13, CCL5, HLA-G, HLA-C, CD86, HLA-DRB1, CCR7, HLA-B, C5AR1, TGFB1, CCR1, C3AR1, R2M, CCL13, NFKB2, CCL1, CXCL1, CCR3, HLA-DOA, and HLA-DRB4.

### Examples

### Identification of cervical cancer biomarkers

Microarray samples of 24 normal and 102 cervical cancer patients from four available studies were pooled and evaluated. High quality microarrays were normalized using the CONOR package from the Bioconductor project. Gene expression profiling was performed using variance-component analysis for accessing most reliable markers, which were subsequently processed by Gene Set Enrichment Analysis.

Of 22.277 markers that were subject to variance-component analysis, eleven markers had low heterogeneity, i.e. a W/T ratio between 0.18 and 0.38. Seven of these markers are induced in all cervical cancer stages these are GINS1, PAK2, DTL, AURKA, PRKDC, NEK2 and CEP55. The other four markers probes are induced in normal cervix P11, EMP1, UPK1A and HSPC159. The inventors performed gene set enrichment analysis (GSEA) of 9.873 markers exhibiting less variability, *i.e.* having a W/T ratio of < 0.75.

Here described is a large-scale variance-component analysis of pooled microarray raw data from four publicly available cervical cancer studies. Inspired by the recent study of Bachtiary et al., the inventors found that intratumor heterogeneity as well as heterogeneity across tumor stages has an influence on the reliability of oncogenic markers.

Therefore statistically as well as clinically relevant gene expression profiles of cervical cancer were developed. In addition to variance-component analysis we GSEA was used to process the resulting profiles (Subramanian et al. 2005). From the results presented here, eleven highly reliable oncogenic markers for distinction between normal and cancerous cervix tissue were derived.

### Cervical cancer microarray studies in GEO

Microarray raw data of four publicly available cervical cancer studies was obtained from Gene Expression Omnibus (GEO, http://www.ncbi.nlm.nih.gov/geo/ (Barrett et al. 2005)).

The following studies were evaluated for high quality arrays.
**Study A:** GSE5787 (Bachtiary et al., on Human Genome U133A Plus 2.0, (Bachtiary et al. 2006)). Purpose of this study was to estimate intratumor heterogeneity in CC patients. As a result, an ANOVA model for the reliability of biomarkers was proposed.
**Study B:** GSE6791 (Pyeon et al., on Human Genome U133A Plus 2.0, (Pyeon et al. 2007)). Purpose of this study was a comparison of gene expression patterns of HPV+ and HPV- head and neck cancers to gene expression patterns of cervical cancers.
**Study C:** GSE9750 (Scotto et al., on Human Genome U133A, (Scotto et al. 2008)). Purpose of this study was to examine the role of the gain on the long arm of chromosome 20 in early stages of CC and to elucidate amplicon functions as well as potential markers.
**Study D:** GSE26511 (Nordhuis et al., on Human Genome U133A Plus 2.0, (Noordhuis et al. 2011)). Purpose of this study was to identify cellular tumor pathways associated with pelvic lymph node status patients with early stages of cervical cancer (Noordhuis et al. 2011).

### Patients

Pooling the above-mentioned four studies the inventors obtained in total microarray data from 102 cervical cancer biopsies and 24 microarray samples of normal cervix. All patients were between 27 and 77 years old (median 42.5). Table 2 shows the number of patients in each assigned tumor stage according to the Federation International of Gynaecology and Obstetrics (FIGO).

### Data processing

An important step before the actual analysis of microarray data comprises the normalization procedure i.e. to largely remove technical variation and enable comparison of the expression values. Normalization algorithms are widely implemented in the open source R based Bioconductor project. R version 2.12 was used for all statistical analysis and computing. Because of the combination of microarray data from four different laboratories and even different manufacturer series, an especially careful approach to normalization has to be considered. First, a quality control was performed using the *Simpleaffy* package, which leads to omitting arrays having an average background intensity of more than 75 and also to the rejection of arrays in which both housekeeping genes (markers) were considered to be off ratio (> 3 fold) (Wilson and Miller 2005).

Additionally an mRNA degradation check was applied. Then, all raw data were pre-processed using quantile normalization of the *RMA* package without background adjustment (Irizarry et al. 2003). Next, the GEO datasets were merged to obtain a patient data matrix using the CONOR package that provides cross-platform normalization of microarray data (Rudy and Valafar 2011).

### Analysis of variance (ANOVA)

The inventors then wished to access the variance-components of all different stages of cervical cancer. Therefore the provided R code was applied on the patient matrix grouped by each distinct FIGO stage of cervical cancer. Hence, in the model W is the variance within a stage, B corresponds to the variance between stages and T is the total variance (T = W + B). The ratio W/T provides a measurement of the intra stages heterogeneity.

### Gene Set Enrichment Analysis (GSEA)

In 2005 Subramanian et al. introduced gene set enrichment analysis (GSEA) (Subramanian et al. 2005). This algorithm tests, whether a gene signature is significantly enriched inside of a collection of various predefined gene sets. Such predefined gene sets comprise markers from biological pathway databases, like KEGG, Biocarta, and Reactome as well as all layers of Gene Ontology terms and gene signatures obtained from scientific publications. The GSEA algorithm calculates an enrichment score that is accompanied by an estimation of significance which is also adjusted for multiple hypothesis testing. A gene set based permutation test using 1.000 permutations was performed, and ranked all markers according to Student's t statistic. Nominal p-value below 0.01 and FDR less than 0.05 were considered as statistically significant. Additionally, the enrichment score was restricted to values above 0.7.

### Results

### Quality assessment and normalization of GEO studies yields a pooled patient data matrix.

Since the quality assessment of microarray data is as important as microarray normalization, each study was inspected individually. In study A (GSE5787) from 33 arrays, only array GSM135260 had an off scale control marker, but the overall quality was sufficient. In study B (GSE6791) only 18 out of 27 possibly useful arrays were actually considered due to quality issues. Even the considered arrays had off scale control markers, however, since the average background and the present signals were in range, these 18 arrays were considered. Study C (GSE9750) had eight out of 57 arrays comprising a very low overall signal compared to the other arrays and thus were rejected from further processing (i.e. GSM246422, GSM246423, GSM246484, GSM246485, GSM246486, GSM246487, GSM246488 and GSM246489). Finally, in study D (GSE26511) all 39 arrays were considered, since these arrays were of recommendable quality on any scale.

Next, all studies were subjected to a second quality check regarding mRNA degradation, and all arrays passed this test. In total, 131 arrays were extracted that met high quality standards. Single samples of stages IA and IIIA as well as three samples from the advanced stage IVB were excluded, since these small sample sizes were not representative.

This left 126 microarrays in total for further processing. Regarding quantity and FIGO stage, the following arrays were used: NC/24; IB/12; IB1/21; IB2/15; IIA/10; IIB/25; and IIIB/19. The different arrays were normalized first within each study by *RMA* quantile normalization without background adjustment (Irizarry et al. 2003). Then, in three subsequent merging steps using the CONOR package all studies were bundled to yield a single pooled patient data matrix.

### Variance component analysis of all available cervical cancer stages yields low heterogeneous markers

The patient data matrix contained 22.277 markers for each of the 126 samples that are representing one normal cervix and six histological conditions of cervical cancer according to FIGO. For accessing markers which are less heterogeneously expressed the inventors performed variance-component analysis. The variance-component analysis of five randomly chosen replicates leads to a steep increase of markers which have a W/T ratio of one. However, a low ratio denotes markers that are less variable inside a specific stage. Clearly, using all available samples is of benefit and led to a distribution that peaks at a W/T ratio of about 0.8. Next the inventors defined a cut-off for accessing only the most reliable markers that are exhibiting less variability inside a stage. Considering markers that had a W/T ratio of 0.75, left 9.873 markers for further processing.

### Clustering highlights low variable markers that are constitutively expressed in all stages.

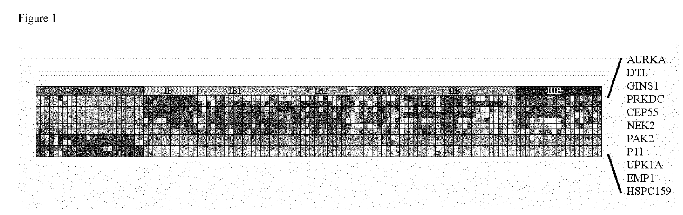

Cluster analysis of 9.873 markers resulting from variance-component analysis yielded eleven markers, which are induced or repressed in cervical cancer, having a W/T ratio between 0.18 and 0.38. Seven of these markers are induced in all cervical cancer stages, these markers are GINS1, PAK2, DTL, AURKA, PRKDC, NEK2 and CEP55. The other markers are repressed in cervical cancer, namely P11, EMP1, UPK1A and HSPC159. Figure 1 displays a heatmap of the biomarkers according to the present invention, depicting induced markers in light gray and repressed markers in dark gray.

### Gene set enrichment analysis of the variance-component derived expression set.

As mentioned above, a total of 9.783 markers exhibit a W/T ratio of less than 0.75, these markers were analyzed using GSEA. Initially, the inventors analyzed the normal phenotype versus all samples from stage I. The majority of the enriched functional gene clusters/gene signatures in cervical cancer are related to DNA maintenance, and also signatures of proliferation and metastasis could be found. In contrast to the gene signature named "Cervical Cancer Proliferation Cluster" (CCPC) which comprised 105 enriched genes which had a p-value and FDR below 1e-4,a signature that was identified earlier by Rosty et al. (Rosty et al. 2005) considered genes exhibiting the highest variance, and thus had 163 genes. Here the inventors could find only genes exhibiting less heterogeneity. A gene set termed "Bidus, Metastasis Up"from to the work of Bidus et al. (Bidus et al. 2006), was found as significantly enriched in the present patient matrix as well

### GSEA derived processes found repeatedly in stages.

Among the most significant and most enriched genbe signatures, "Wilensky, Response to Darapladib" (ES 0.90) and "Gaurnier, PSMD4 Targets" (ES 0.89) were found in stage IB1. These signatures are repeatedly enriched in stage IIA yielding enrichment scores (ES) of 0.84 and ES 0.76. Then, the Gaurnier signature was found once again enriched in stage IIB, having an ES of 0.80. Wilensky et al. acquired a gene expression signature that is down-regulated after treatment with lipoprotein-associated phospholipase A2 (Lp-PLA2) inhibitor darapladib.

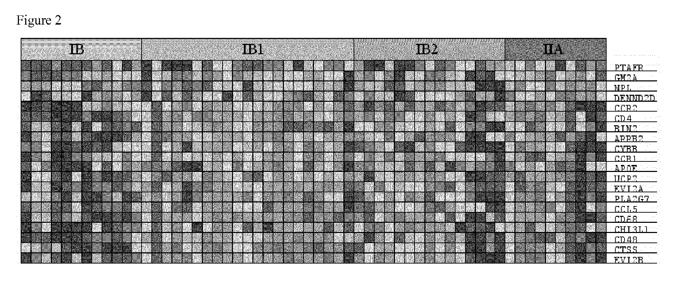

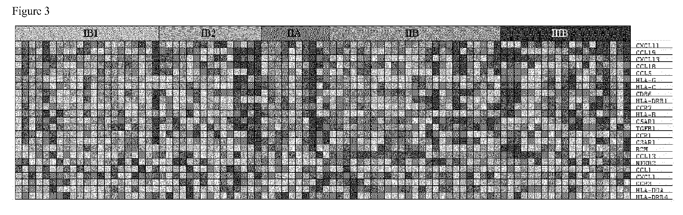

In stage IB1 and stage IIA the inventors found 20 genes of the Wilensky signature up-regulated, having significant enrichment scores (p-value and FDR < 1e-4) as shown in Figure 2. The complete Gaurnier signature consists of 59 genes, of which the inventors could find 23 (39 %) in our data that are yielding significant enrichment scores in stage IB1, IIA and IIB as shown in Figure 3. However, a significant enrichment of a fraction of the complete signature was found (p-value and FDR < 1e-4). It was shown that the activation of anti-tumoral, MMP9 secreting monocytes is induced by treatment using angiocidin, the protein product of PSMD4 (Gaurnier-Hausser et al. 2008).

### Induction in stages of cancer/HPV infection

The gene set enrichment analysis of variance component derived genes, yields a characterization of transcriptional programs in each cervical cancer stage. While the approach produces overlap with gene signatures from different studies, it only highlights robust signatures, omitting high variable and therefore actually unreliable genes.

### Literature as cited

Bachtiary B, Boutros PC, Pintilie M, Shi W, Bastianutto C, Li J-H, Schwock J, Zhang W, Penn LZ, Jurisica I, Fyles A, Liu F-F (2006) Gene expression profiling in cervical cancer: an exploration of intratumor heterogeneity. Clin Cancer Res 12:5632-5640.
Barrett T, Suzek TO, Troup DB, et. al. (2005) NCBI GEO: mining millions of expression profiles - database and tools. Nucleic Acids Res 33:D562-566.
Bidus MA, Risinger JI, Chandramouli GV, Dainty LA, Litzi TJ, Berchuck A, Barrett JC, Maxwell GL (2006) Prediction of lymph node metastasis in patients with endometrioid endometrial cancer using expression microarray. Clin Cancer Res 12 (1):83-88.
Browne EP, Wing B, Coleman D, Shenk T (2001) Altered cellular mRNA levels in human cytomegalovirus-infected fibroblasts: viral block to the accumulation of antiviral mRNAs. Journal of virology 75 (24):12319-12330.
Flechner SM, Kurian SM, Head SR, Sharp SM, Whisenant TC, Zhang J, Chismar JD, Horvath S, Mondala T, Gilmartin T, Cook DJ, Kay Sa, Walker JR, Salomon DR (2004) Kidney transplant rejection and tissue injury by gene profiling of biopsies and peripheral blood lymphocytes. Am J Transplant 4:1475-1489.
Gaurnier-Hausser A, Rothman VL, Dimitrov S, Tuszynski GP (2008) The novel angiogenic inhibitor, angiocidin, induces differentiation of monocytes to macrophages. Cancer Res 68 (14):5905-5914.
Insinga A, Monestiroli S, Ronzoni S, Gelmetti V, Marchesi F, Viale A, Altucci L, Nervi C, Minucci S, Pelicci PG (2005) Inhibitors of histone deacetylases induce tumor-selective apoptosis through activation of the death receptor pathway. Nat Med 11:71-76.
Irizarry R, Hobbs B, Collin F, et. al. (2003) Exploration, normalization, and summaries of high density oligonucleotide array probe level data. Biostatistics 4:249-264.
Jemal A, Bray F, Center MM, Ferlay J, Ward E, Forman D (2011) Global cancer statistics. CA: a cancer journal for clinicians 61 (2):69-90.
Laneve P, Gioia U, Ragno R, Altieri F, Di Franco C, Santini T, Arceci M, Bozzoni I, Caffarelli E (2008) The tumor marker human placental protein 11 is an endoribonuclease. J Biol Chem 283 (50):34712-34719.
Lowy DR, Schiller JT (2006) Prophylactic human papillomavirus vaccines. J Clin Invest 116 (5):1167-1173.
Noordhuis MG, Fehrmann RS, Wisman GB, Nijhuis ER, van Zanden JJ, Moerland PD, Ver Loren van Themaat E, Volders HH, Kok M, ten Hoor KA, Hollema H, de Vries EG, de Bock GH, van der Zee AG, Schuuring E (2011) Involvement of the TGF-beta and beta-catenin pathways in pelvic lymph node metastasis in early-stage cervical cancer. Clin Cancer Res 17 (6):1317-1330.
Pyeon D, Newton Ma, Lambert PF, den Boon Ja, Sengupta S, Marsit CJ, Woodworth CD, Connor JP, Haugen TH, Smith EM, Kelsey KT, Turek LP, Ahlquist P (2007) Fundamental differences in cell cycle deregulation in human papillomavirus-positive and human papillomavirus-negative head/neck and cervical cancers. Cancer Res 67:4605-4619.
Rosty C, Sheffer M, Tsafrir D, Stransky N, Tsafrir I, Peter M, de Cremoux P, de La Rochefordiere A, Salmon R, Dorval T, Thiery JP, Couturier J, Radvanyi F, Domany E, Sastre-Garau X (2005) Identification of a proliferation gene cluster associated with HPV E6/E7 expression level and viral DNA load in invasive cervical carcinoma. Oncogene 24 (47):7094-7104.
Rozanov DV, Savinov AY, Williams R, Liu K, Golubkov VS, Krajewski S, Strongin AY (2008) Molecular signature of MT1-MMP: transactivation of the downstream universal gene network in cancer. Cancer Res 68 (11):4086-4096.
Rudy J, Valafar F (2011) Empirical comparison of cross-platform normalization methods for gene expression data. BMC Bioinformatics 12 (1):467.
Scotto L, Narayan G, Nandula SV, Arias-Pulido H, Subramaniyam S, Schneider A, Kaufmann AM, Wright JD, Pothuri B, Mansukhani M, Murty VV (2008) Identification of copy number gain and overexpressed genes on chromosome arm 20q by an integrative genomic approach in cervical cancer: potential role in progression. Genes, chromosomes & cancer 47 (9):755-765.
Slebos RJ, Yi Y, Ely K, Carter J, Evjen A, Zhang X, Shyr Y, Murphy BM, Cmelak AJ, Burkey BB, Netterville JL, Levy S, Yarbrough WG, Chung CH (2006) Gene expression differences associated with human papillomavirus status in head and neck squamous cell carcinoma. Clin Cancer Res 12 (3 Pt 1):701-709.
Subramanian A, Tamayo P, Mootha VK, et. al. (2005) Gene set enrichment analysis: a knowledge-based approach for interpreting genome-wide expression profiles. Proc Natl Acad Sci USA 102:15545-15550.
Ueno M, Itoh M, Kong L, Sugihara K, Asano M, Takakura N (2005) PSF1 is essential for early embryogenesis in mice. Mol Cell Biol 25 (23):10528-10532.
Wieland S, Thimme R, Purcell RH, Chisari FV (2004) Genomic analysis of the host response to hepatitis B virus infection. Proc Natl Acad Sci USA 101 (17):6669-6674.
Wilensky RL, Shi Y, Mohler ER, 3rd, Hamamdzic D, Burgert ME, Li J, Postle A, Fenning RS, Bollinger JG, Hoffman BE, Pelchovitz DJ, Yang J, Mirabile RC, Webb CL, Zhang L, Zhang P, Gelb MH, Walker MC, Zalewski A, Macphee CH (2008) Inhibition of lipoprotein-associated phospholipase A2 reduces complex coronary atherosclerotic plaque development. Nat Med 14 (10):1059-1066.
Wilson CL, Miller CJ (2005) Simpleaffy: a BioConductor package for Affymetrix Quality Control and data analysis. Bioinformatics 21:3683-3685.
Wolfer A, Ramaswamy S (2011) MYC and metastasis. Cancer Res 71:2034-2037
Zhang J, Cao W, Xu Q, Chen WT (2011) The expression of EMP1 is downregulated in oral squamous cell carcinoma and possibly associated with tumour metastasis. Journal of clinical pathology 64 (1):25-29.

## Claims

1. A method for diagnosing cervical cancer in a subject having an infection with the human papilloma virus (HPV) and/or for the detection of a persistent HPV infection, the method comprising detecting the expression and/or biological activity of at least one of the diagnostic markers or fragments thereof selected from the group consisting of PAK2, NEK2, AURKA, PRKDC, DTL, CEP55, GINS1, P11, EMP1, UPK1A, and HSPC159 in a sample obtained from said subject.

2. The method according to claim 1, wherein said detecting step is carried out by means of the determination of the gene expression of the at least one diagnostic marker in said isolated sample, such as, for example, by means of the determination of the transcripts of the diagnostic markers and/or of the proteins encoded by the diagnostic markers.

3. The method according to claim 1 or 2, wherein an increase of the expression and/or biological activity of at least one of the diagnostic markers selected from the group consisting of PAK2, NEK2, AURKA, PRKDC, DTL, CEP55, GINS1, and/or a decrease of the expression and/or biological activity of at least one of the diagnostic markers selected from the group consisting of P11, EMP1, UPK1A and HSPC159 is indicative for cervical cancer and/or for a persistent HPV infection.

4. The method according to any of claims 1 to 3, wherein said diagnosing furthermore comprises determining the expression and/or biological activity of a reference marker, such as, for example a housekeeping gene selected from at least one of N-acylsphingosine amide hydrolase 1, HIG1 domain family member 1A (hypoxia-inducible gene 1), and stress-associated endoplasmic reticulum protein 1.

5. The method according to any of claims 1 to 4, wherein said diagnosing furthermore comprises determining the stage of the cervical cancer and/or persistent HPV infection, based on said at least one diagnostic marker, such as, for example, very early stages of the cancer, like IB, IB1 and/or IB2.

6. The method according to any of claims 1 to 5, wherein said determining comprises a method selected from subtractive hybridization, microarray analysis, mutation analysis by DNA sequencing, qPCR, ELISA, enzymatic activity tests, cell viability assays, for example an MTT assay, phosphoreceptor tyrosine kinase assays, phospho-MAPK arrays and proliferation assays, for example the brdU assay, proteomics, and mass spectrometry.

7. The method according to any of claims 1 to 6, wherein said sample is a tissue or body fluid sample of a human, such as, for example, a biopsy, a cervical cell sample and/or a Papanicolaou smear.

8. A method for monitoring the treatment of a subject having cervical cancer and/or a persistent HPV infection, comprising the steps of
a) providing an anti-cervical cancer and/or anti-HPV infective treatment to said subject, and
b) performing a diagnosis according to any of claims 1 to 7 on a sample obtained from said subject, and
c) optionally, repeating steps a) and b), if required.

9. The method according to claim 8, wherein said treatment comprises the administration of a phospholipase inhibitor, such as, for example, darapladib, and/or an angiogenic inhibitor, such as, for example, angiocidin.

10. The method according to claim 8 or 9, further comprising the step of generating an expression profile and/or activity profile based on said diagnostic markers, wherein said profile consists of markers that predict response to specific treatments, in particular patient-specific treatments.

11. A method for identifying an anti-cervical cancer and/or anti-HPV infective agent, comprising the steps of
a) contacting a sample obtained from a subject having cervical cancer and/or a persistent HPV infection with at least one compound which potentially modifies the expression and/or biological activity of at least one diagnostic marker selected from the group consisting of PAK2, NEK2, AURKA, PRKDC, DTL, CEP55, GINS1, P11, EMP1, UPK1A, and HSPC159, and
b) identifying a compound that modifies the expression and/or biological activity of said at least one diagnostic marker.

12. The method according to claim 11, wherein said compound is selected from a phospholipase inhibitor and/or an angiogenic inhibitor.

13. A panel of biomarkers for diagnosing cervical cancer in a subject having an infection with the human papilloma virus (HPV) and/or for the detection of a persistent HPV infection, wherein said panel comprises at least two of the diagnostic markers or fragments thereof selected from the group consisting of PAK2, NEK2, AURKA, PRKDC, DTL, CEP55, GINS1, P11, EMP1, UPK1A, and HSPC159.

14. Darapladib and/or angiocidin for the treatment of cervical cancer and/or HPV infection.

15. A diagnostic kit comprising materials for performing a method according to any of claims 10 to 14 in one or separate containers, preferably comprising at least one biomarker-specific antibody, optionally together with auxiliary agents and/or instructions for performing said method.
